(19) Europäisches Patentamt
European Patent Office
Office européen des brevets

(11) Publication number: **0 451 772 A1**

(12) **EUROPEAN PATENT APPLICATION**

(21) Application number: **91105567.1**

(22) Date of filing: **09.04.91**

(51) Int. Cl.[5]: **C07D 409/04**, A61K 31/445, //(C07D409/04,333:00,211:00), (C07D409/04,335:00,211:00), (C07D409/04,337:00,211:00)

(30) Priority: **11.04.90 JP 93968/90**
**16.04.90 JP 97522/90**

(43) Date of publication of application:
**16.10.91 Bulletin 91/42**

(84) Designated Contracting States:
**AT BE CH DE ES FR GB GR IT LI NL SE**

(71) Applicant: **HOKURIKU PHARMACEUTICAL CO., LTD.**
**1-Chome, 3-14, Tatekawacho, Katsuyama-shi**
**Fukui-ken(JP)**

(72) Inventor: **Ito, Yasuo**
**Moto-machi 3-chome**
**Katsuyama-shi, Fukui-ken(JP)**
Inventor: **Kato, Hideo**
**11-37, Motomachi 1-chome**
**Katsuyama-shi, Fukui-ken(JP)**
Inventor: **Koshinaka, Eiichi**
**6-3, Asahi-cho 2-chome**
**Katsuyama-shi, Fukui-ken(JP)**
Inventor: **Ogawa, Nobuo**
**6-5, Asahi-cho 2-chome**
**Katsuyama-shi, Fukui-ken(JP)**
Inventor: **Nishino, Hiroyuki**
**23-14, Matta , Arado-cho**
**Katsuyama-shi, Fukui-ken(JP)**
Inventor: **Sakaguchi, Jun**
**12-6-2, Inokeya**
**Katsuyama-shi, Fukui-ken(JP)**

(74) Representative: **Werner, Hans-Karsten, Dr. et al**
**Deichmannhaus am Hauptbahnhof**
**W-5000 Köln 1(DE)**

(54) **Piperidine compounds, method for preparation thereof, and a pharamceutical composition comprising the same.**

(57) Novel piperidine compounds represented by the following formula (I):

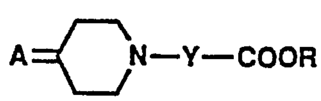

(I)

wherein Y represents an alkylene group having 1 to 7 carbon atoms; A is a group represented by formula I-a or formula I-b:

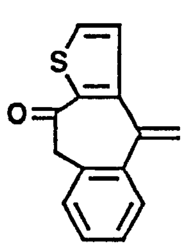

(1-a)

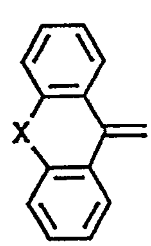

(I-b)

EP 0 451 772 A1

wherein X represents $-CH_2-S-$ or -S-; and R represents a hydrogen atom or a lower alkyl group with a proviso that A is a group represented by formula I-a, or R represents a hydrogen atom with a proviso that A is a group represented by formula I-b and pharmacologically acceptable salts thereof are disclosed. Also disclosed are a method for preparing the same; a pharmaceutical composition comprising the same; an antiallergic agent and an agent for bronchial asthma comprising the same; and a method for treatment of an allergic disease or bronchial asthma comprising the step of administering the same.

BACKGROUND OF THE INVENTION

Field of the Invention

The present invention relates to novel piperidine compounds and pharmacologically acceptable salts thereof which have an antihistaminic and antiallergic activity and are useful for the treatment of, for example, bronchial asthma, allergic rhinitis, dermatosis, and urticaria, and to the method for preparation thereof.

The present invention also relates to a pharmaceutical composition comprising the effective amount of the same.

Description of the Prior Art

Several piperidine compounds have been found to be useful as an antihistaminic agent and an antiallergic agent. An example of the piperidine compound is ketotifen (The Merck Index, 11th edition, 5187: 4,9-dihydro-4-(1-methyl-4-piperidinylidene)-10H-benzo[4,5]cyclohepta[1,2-b]thiophen-10-one) represented by the following formula:

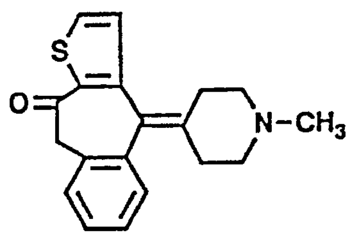

which is widely used clinically for the treatment of such ailments as allergic diseases and bronchial asthma.

A large number of antihistaminic agents have been developed so far and are used for the treatment of, for example, allergic dermatosis or rhinitis. However, adverse reactions of a central inhibitory action such as sleepiness or sedation are found to be a great problem with these known agents. In addition, an anticholinergic action which is considered to be one of the possible reasons for hydrodipsia or mydriasis is another undesired adverse reaction of the antihistaminic agents. Various kinds of research have been conducted to solve the above problems, however, presently available antihistaminic agents are insufficient from a clinical point of view.

SUMMARY OF THE INVENTION

An object of the present invention is to provide novel compounds having an excellent antihistaminic activity as well as excellent antiallergic activity.

Another object of the present invention is to provide novel compounds which extensively eliminate undesired adverse reactions such as a central inhibitory action when administered for the treatment of such ailments as bronchial asthma and allergic diseases.

A further object of the present invention is to provide a method for preparation of said compounds. Yet another object is to provide a pharmaceutical composition comprising said compounds which is useful for the treatment of such ailments as allergic diseases and bronchial asthma.

The inventors of the present invention have conducted various studies to achieve the foregoing objects and found that the objects can be effectively attained by providing novel piperidine compounds of the present invention having excellent antihistaminic and antiallergic activities.

In accordance with the above objects, the present invention provides piperidine compounds represented by the following formula (I):

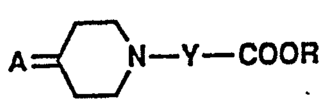

(I)

wherein Y represents an alkylene group having 1 to 7 carbon atoms; A is a group represented by the

following formula I-a:

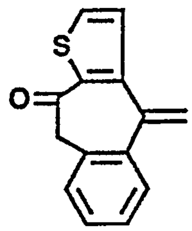

(I-a)

or A is a group represented by the following formula I-b:

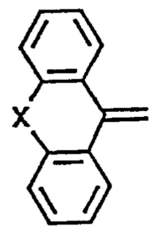

(I-b)

wherein X represents -CH, -S- or -S-; and R represents a hydrogen atom or a lower alkyl group with a proviso that A is a group represented by formula I-a, or R represents a hydrogen atom with a proviso that A is a group represented by formula I-b; and pharmacologically acceptable salts of the above compounds.

In accordance with another embodiment of the present invention, the present invention provides a process for preparing piperidine compounds of formula (I).

In accordance with yet another embodiment, the present invention provides an antihistaminic agent and antiallergic agent comprising an effective amount of a piperidine compound of formula (I).

In accordance with a further embodiment, the present invention provides a pharmaceutical composition for the treatment of an allergic disease comprising an effective amount of a piperidine compound of formula (I).

The invention also provides a method of treating an allergic disease comprising the step of administering to a mammal an effective amount of a piperidine compound of formula (I).

Further objects, features and advantages of the present invention will become apparent from the Description of the Preferred Embodiments which follows, when read in light of the attached Examples.

DETAILED DESCRIPTION OF THE PREFERRED EMBODIMENTS

The present invention provides piperidine compounds represented by the following formula (I):

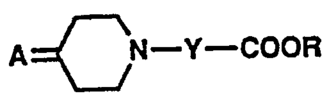

(I)

wherein Y represents an alkylene group having 1 to 7 carbon atoms; A is a group represented by the following formula I-a:

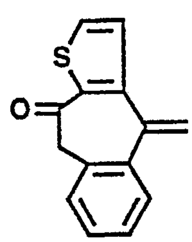

(I-a)

or A is a group represented by the following formula I-b:

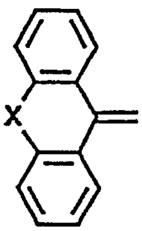

(I-b)

wherein X represents -CH,-S- or -S-; and R represents a hydrogen atom or a lower alkyl group with a proviso that A is a group represented by formula I-a, or R represents a hydrogen atom with a proviso that A is a group represented by formula I-b; and pharmacologically acceptable salts of the above compounds.

The present invention also provides a process for preparing the piperidine compounds of formula (I), and a pharmaceutical composition comprising an effective amount of the same together with a pharmaceutically acceptable carrier or coating.

In the above formula (I), the lower alkyl group represented by R may be, for example, methyl, ethyl, n-propyl, isopropyl, n-butyl, isobutyl, or tert-butyl group.

Preferred examples of the present invention include:

4-(4,9-dihydro-10-oxo-10H-benzo[4,5]cyclohepta[1,2-b]thiophen-4-ylidene)-1-piperidineacetic acid;
4-(4,9-dihydro-10-oxo-10H-benzo[4,5]cyclohepta[1,2-b]thiophen-4-ylidene)-1-piperidinepropionic acid;
4-(4,9-dihydro-10-oxo-10H-benzo[4,5]cyclohepta[1,2-b]thiophen-4-ylidene)-1-piperidinebutyric acid;
4-(4,9-dihydro-10-oxo-10H-benzo[4,5]cyclohepta[1,2-b]thiophen-4-ylidene)-1-piperidinevaleric acid;
4-(4,9-dihydro-10-oxo-10H-benzo[4,5]cyclohepta[1,2-b]thiophen-4-ylidene)-1-piperidinecaproic acid;
4-(9H-thioxanthen-9-ylidene)-1-piperidinepropionic acid;
4-(9H-thioxanthen-9-ylidene)-1-piperidinebutyric acid;
4(9H-thioxanthen-9-ylidene)-1-piperidinevaleric acid;
4-(dibenzo[b,e]thiepin-11(6H)-ylidene)-1-piperidineacetic acid;
4-(dibenzo[b,e]thiepin-11(6H)-ylidene)-1-piperidinepropionic acid;
4-(dibenzo[b,e]thiepin-11(6H)-ylidene)-1-piperidinebutyric acid;
4-(dibenzo[b,e]thiepin-11(6H)-ylidene)-1-piperidinevaleric acid;
4-(dibenzo[b,e]thiepin-11(6H)-ylidene)-1-piperidinecaproic acid;
4-(dibenzo[b,e]thiepin-11(6H)-ylidene)-1-piperidineheptanoic acid;
4-(dibenzo[b,e]thiepin-11(6H)-ylidene)-1-piperidineoctanoic acid;
ethyl 4-(4,9-dihydro-10-oxo-10H-benzo[4,5]cyclohepta[1,2-b]thiophen-4-ylidene)-1-piperidineacetate;
ethyl 4-(4,9-dihydro-10-oxo-10H-benzo[4,5]cyclohepta[1,2-b]thiophen-4-ylidene)-1-piperidinepropionate;
tert-butyl 4-(4,9-dihydro-10-oxo-10H-benzo[4,5]cyclohepta[1,2-b]thiophen-4-ylidene)-1-piperidinepropionate;
ethyl 4-(4,9-dihydro-10-oxo-10H-benzo[4,5]cyclohepta[1,2-b]thiophen-4-ylidene)-1-piperidinebutyrate;
ethyl 4-(4,9-dihydro-10-oxo-10H-benzo[4,5]cyclohepta[1,2-b]thiophen-4-ylidene)-1-piperidinevalerate; and
methyl 4-(4,9-dihydro-10-oxo-10H-benzo[4,5]cyclohepta[1,2-b]thiophen 4-ylidene)-1-piperidinecaproate.

The compounds of the present invention represented by the above formula (I) may be converted to pharmacologically acceptable salts, if desired, and may then be reconverted to produce the free compound from the obtained salts.

The pharmacologically acceptable salts of the compounds of the present invention represented by formula (I) may be acid addition salts or alkali addition salts. Examples of the acid addition salts include mineral acid addition salts such as, for example, hydrochloride, hydrobromide, sulfate, nitrate, and phosphate, and organic acid salts such as, for example, acetate, maleate, fumarate, malate, citrate, oxalate, lactate, and tartarate. Examples of the alkali addition salts include metal salts such as, for example, sodium, potassium, and calcium salt, and organic alkali salts such as, for example, ammonium salts, methylamine, ethylamine, dimethylamine, triethylamine, ethanolamine, piperidine, and piperazine salts.

The novel piperidine compound of the present invention represented by the above formula (I) can be prepared by reacting a compound represented by the following general formula (II):

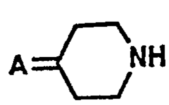

(II)

wherein A is a group represented by the following formula I-a:

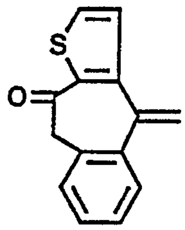

(I-a)

or A is a group represented by the following formula I-b:

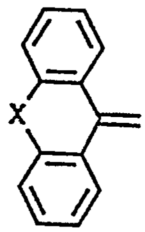

(I-b)

wherein X represents $-CH_2$, -S- or -S-, with a compound represented by the following formula (III):

Z-Y-COOR (IIIa) or $CH_2=CH-COOR$ (IIIb)

wherein Y represents an alkylene group having 1 to 7 carbon atoms; R represents a hydrogen atom or a lower alkyl group with a proviso that A is a group represented by formula I-a, or R represents a hydrogen atom with a proviso that A is a group represented by formula I-b; and z represents a halogen atom, in a solvent or without a solvent in the presence or absence of a base as a dehydrohalogenating agent, and followed by hydrolysis by using an acid or a base, if necessary.

Any inert solvent may be used in the alkylation process of the present invention. Examples of the inert solvent include benzene, toluene, tetrahydrofuran, dioxane, acetone, acetonitrile, methanol, ethanol, isopropanol, n-butanol, dimethyl sulfoxide, and N,N-dimethylformamide. Examples of the base used in the process of the present invention include potassium carbonate, sodium carbonate, pyridine, and triethylamine. The reaction may be carried out at a temperature of from 0 to 200 °C.

For the hydrolysis process, an acid such as, for example, hydrochloric acid or sulfuric acid, or a base such as, for example, sodium hydroxide, potassium hydroxide, potassium carbonate, sodium carbonate, or sodium bicarbonate may be used. A solvent used in the hydrolysis process may be, for example, water, methanol, ethanol, acetone, or tetrahydrofuran, and the hydrolysis may be carried out at a temperature of from 0 to 100 °C.

The compounds represented by the above formula (II), used as starting materials for the above process, are known compounds disclosed in the Japanese Unexamined Patent Publication Nos. 18478/1975 and 106573/1986 and Helvetica Chimica Acta 59, 866(1976), which can be readily prepared by the following process:

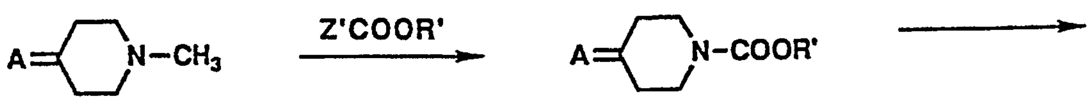


(II)

wherein, A represents the same as that defined above, Z' represents a halogen atom, and R' represents a lower alkyl group.

The novel piperidine compounds of the present invention represented by the above formula (I) and the pharmacologically acceptable salts of the compounds have excellent antihistaminic and antiallergic activities, and thus are quite useful for the treatment of allergic diseases, such as, for example, bronchial asthma, allergic rhinitis, dermatosis, and urticaria.

The piperidine compounds of the present invention and their pharmacologically acceptable salts may be administered orally or parenterally to a patient as a pharmaceutical composition which comprises an effective amount of said compound or said salt together with a pharmaceutically acceptable carrier or coating.

The pharmaceutical composition suitable for oral administration may be, for example, tablet, capsule, powder, subtilised granule, granule, solution, or syrup. The pharmaceutical composition suitable for parenteral administration may be injection, suppository, inhalant, eye drop, nasal drop, ointment, or cataplasm. The pharmaceutically acceptable carrier or coating used for the preparation of the pharmaceutical composition may be, for example, excipient, disintegrant or agent for accelerating disintegration, binder, lubricant, coating agent, pigment, diluent, base, solubilizing agent or solubilizer, isotonicity, pH adjusting agent, stabilizer, propellant, and adhesive.

For the preparation of the pharmaceutical composition suitable for oral administration, dermal administration, or mucosal application, the coating or carrier may comprise the following: an excipient such as, for example, glucose, lactose, D-mannitol, starch, or crystalline cellulose; a disintegrant or an agent for accelerating disintegration such as, for example, carboxymethylcellulose, starch, or calcium carboxymethylcellulose; a binder such as, for example, hydroxypropylcellulose, hydroxypropylmethylcellulose, polyvinylpyrrolidone, or gelatin; a lubricant such as, for example, magnesium stearate or talc; a coating agent such as, for example, hydroxypropylmethylcellulose, sucrose, polyethylene glycol, or titanium oxide; a base such as, for example, petrolatum, liquid paraffin, polyethyleneglycol, gelatin, kaolin, glycerin, purified water, or hard fat; a propellant such as, for example, fron, diethylether, or compressed gas; an adhesive such as, for example, sodium polyacrylate, polyvinylalcohol, methylcellulose, polyisobutylene, or polybutene; or a base sheet such as, for example, cloth or plastic sheet. The pharmaceutical composition suitable for injection may comprise the following: a solubilizing agent or a solubilizer, e.g., distilled water for injection, saline, or propylene glycol which is useful for an aqueous composition or a composition for preparing aqueous solution before use; an isotonicity agent such as, for example, glucose, sodium chloride, D-mannitol, or glycerin; and a pH adjusting agent such as, for example, an inorganic or organic acid or an inorganic or organic base.

The dose of the pharmaceutical composition of the present invention for an adult patient may generally be from about 1 to 500 mg per day for oral administration, which may be increased or decreased depending on the age or conditions of the patient to be treated.

Action

The following example shows the excellent effectiveness of the compounds of the present invention. The results of 48 hr homologous passive cutaneous anaphylaxis (PCA) in rats with the monitoring of antiallergic activity and the results of potentiation of hexobarbital-induced anesthesia in mice with the monitoring of central nervous depressive activity are summarized in Table 1. The following compound was used as the reference compound. Reference compound : Ketotifen-Fumarate

1. 48 hr homologous passive cutaneous anaphylaxis (PCA) in rats

a) Preparation of DNP-As and rat anti-DNP-As serum

Ascaris extract coupled with 2, 4-dinitrophenyl group (DNP-As) was prepared by the method of Koda et al. (Folia pharmacol. japon., 78, 319-334, 1981) and anti-DNP-As containing IgE antibody serum was prepared by the method of Tada and Okumura (J. Immunol., 106, 1019-1025, 1971). The PCA titer of the antiserum was estimated to be 1:128 by 48 hr PCA in rats.

b) 48 hr homologous passive cutaneous anaphylaxis (PCA) in rats

Male Wistar rats weighing 160 to 200 g were sensitized passively by intradermal injection, in the back, of 0.05 ml of anti-DNP-As serum diluted 21-fold with saline. After 48 hr, the animals (18-20 hr fasted) were given iv. 0.5 ml of 1% Evans blue solution containing 1 mg of DNP-As. After an additional 30 min, the animals were killed by stunning and the skins were removed. The intensity of the response was evaluated by assaying the amount of dye leaked according to the method of Katayama et al. (Microbiol. Immunol., 22, 89-101, 1978). The percent inhibition of PCA was calculated using the following formula:

$$\text{Percent inhibition} = \frac{\text{Amount of dye leaked with control} - \text{Amount of dye leaked with test compound}}{\text{Amount of dye leaked with control}} \times 100$$

Test compounds were given orally in a dose of 1 mg/kg 1 hr prior to challenge with antigen. As a control, 5 ml/kg of vehicle (0.5 % CMC) alone were given in a similar manner.

The results are shown in Table 1.

2. Potentiation of hexobarbital-induced anesthesia in mice

The loss of righting reflex induced by hexobarbital was used as an index of anesthesia. Groups of eight male ddY mice (20-24 hr fasted) weighing 19 to 27 g were treated orally with the test compounds (30 mg/kg) or vehicle. Thirty min later, 80 mg/kg of hexobarbital sodium were injected i.p. to the animals and the duration of loss of righting reflex was observed. The percent increase of sleeping time was calculated using the following formula:

$$\text{Percent increase} = \frac{\text{Sleeping time of test compound} - \text{Sleeping time of control}}{\text{Sleeping time of control}} \times 100$$

The results are listed in Table 1.

Table 1

| Test compound | the percent inhibition of PCA in rats (%, 1 mg/kg, p.o.) | the percent increase of hexobarbital-induced anesthesia in mice (%, 30 mg/kg, p.o.) |
|---|---|---|
| Example 5 | 75 | 24 |
| Example 6 | 77 | 26 |
| Example 8 | 71 | 27 |
| Example 10 | 69 | 19 |
| Example 13 | 58 | 30 |
| Example 14 | 63 | 19 |
| Example 15 | 57 | 33 |
| Example 18 | 96 | 49 |
| Reference compound | 56 | 90 |

The present compounds exhibited more potent of antiallergic activity and less potent of central nervous depressive activity than the reference compound.

EXAMPLES

The present invention will be further illustrated by the following References and Examples. The examples are given by way of illustration only and all not be construed as limiting.

Reference 1

Ethyl 4-(9H-Thioxanthen-9-ylidene)-1-piperidinecarboxylate

A mixture of 18.0 g of 1-methyl-4-(9H-thioxanthen-9-ylidene) piperidine and 132.8 g of ethyl chlorocarbonate in 100 ml of toluene was refluxed for 41.5 hrs. After cooling, the reaction mixture was washed with hydrochloric acid and water, dried and concentrated. The residue was solidified by treatment of isopropyl ether to give 18.2 g of pale brown crystals, which were recrystallized from ethanol to give slightly yellowish brown plates, mp 132-134 °C.

Analysis for $C_{21}H_{21}NO_2S$:

| | |
|---|---|
| Calculated | C, 71.76; H, 6.02; N, 3.99. |
| Found | C, 71.51; H, 6.01; N, 3.74. |

Reference 2

Ethyl 4-(Dibenzo[b,e]thiepin-11(6H)-ylidene)-1-piperidinecarboxylate

A mixture of 8.95 g of 4-(dibenzo[b,e]thiepin-11(6H)-ylidene)-1-methylpiperidine and 50 ml of ethyl chlorocarbonate was refluxed for 5 hrs. After cooling, the insoluble precipitates were filtered off and the filtrate was concentrated. The residue was purified by column chromatography on alumina (eluent: methylene chloride) to give 7.86 g of pale yellow liquid.

| | |
|---|---|
| Mass spectrum m/z: | 365 (M + ). |
| IR spectrum $\nu$ (liq) $cm^{-1}$: | 1698 (COO). |
| NMR spectrum $\delta$ ($CDCl_3$) ppm: | 1.25(3H, t, J=7Hz), 2.00-2.65(4H, m), 2.95-3.97(4H, m), 3.39(1H, d, J=13.5Hz), 4.14(2H, q, J=7Hz), 4.89(1H, d, J=13.5Hz), 6.75-7.50-(8H, m). |

Reference 3

4-(9H-Thioxanthen-9-ylidene)piperidine Hydrochloride

A mixture of 17.5 g of ethyl 4-(9H-thioxanthen-9-ylidene)-1-piperidinecarboxylate and 14.0 g of potassium hydroxide in 90 ml of n-butanol was refluxed for 2 hrs and concentrated. Water was added to the residue and extracted with toluene. The extract was washed with water, dried and concentrated. The residue was solidified by treatment of isopropyl ether to give 10.7 g of pale brown solid, which was converted to the hydrochloride in the usual manner and recrystallized from a mixture of methanol and ether to give pale yellow plates, mp > 300 °C.

Analysis for $C_{18}H_{17}NS \cdot HCl$:

| | |
|---|---|
| Calculated | C, 68.45; H, 5.74; N, 4.43. |
| Found | C, 68.42; H, 5.71; N, 4.20. |

The compound of Reference 4 was prepared in the same manner described in Reference 3.

Reference 4

4-(Dibenzo[b,e]thiepin-11(6H)-ylidene)piperidine Hydrochloride

Pale brown plates, mp 293-295 °C(dec.) (EtOH-$Et_2O$).

Analysis for $C_{19}H_{19}NS \cdot HCl$:

| | |
|---|---|
| Calculated | C, 69.18; H, 6.11; N, 4.25. |
| Found | C, 68.97; H, 6.34; N, 4.38. |

Example 1

Ethyl 4-(4,9-Dihydro-10-oxo-10H-benzo[4,5]cyclohepta[1,2-b]thiophen-4-ylidene)-1-piperidinebutyrate Hydrochloride

A mixture of 1.50 g of 4,9-dihydro-4-(4-piperidinylidene)-10H-benzo[4,5]cyclohepta[1,2-b]thiophen-10-one, 1.48 g of ethyl 4-bromobutyrate and 1.00 g of triethylamine in 8 ml of N,N-dimethylformamide was stirred at 60 °C for 2 hrs. After cooling, water was added to the reaction mixture and extracted with ethyl acetate. The extract was washed with water, dried and concentrated. The residue was purified by column chromatography on silica gel [eluent: chloroform-methanol (50:1)] to give 2.21 g of pale yellow viscous liquid, which was converted to the hydrochloride in the usual manner to give 1.86 g of colorless crystals. The crude hydrochloride was recrystallized from a mixture of ethanol and ether to give colorless needles, mp 208-215 °C (dec.).

Analysis for $C_{24}H_{27}NO_3S \cdot HCl$:

| | |
|---|---|
| Calculated | C, 64.63; H, 6.33; N, 3.14. |
| Found | C, 64.67; H, 6.21; N, 3.08. |

The compounds of Example 2 to 4 were prepared in the same manner described in Example 1.

Example 2
Ethyl 4-(4,9-Dihydro-10-oxo-10H-benzo[4,5]cyclohepta[1,2-b]thiophen-4-ylidene)-1-piperidineacetate

Pale yellow viscous liquid.

| | |
|---|---|
| Mass spectrum m/z: | 381 ($M^+$). |
| IR spectrum $\nu$ (liq) $cm^{-1}$: | 1738 (COO), 1656 (CO). |
| NMR spectrum $\delta$ ($CDCl_3$) ppm: | 1.26(3H, t, J=7Hz), 2.15-3.10(8H, m), 3.24(2H, s), 3.74(1H, d, J=13.5Hz), 4.18(2H, q, J=7Hz), 4.22(1H, d, J=13.5Hz), 7.03(1H, d, J=5Hz ), 7.10-7.40 (4H, m), 7.52(1H, d, J=5Hz). |

Example 3
Ethyl 4-(4,9-Dihydro-10-oxo-10H-benzo[4,5]cyclohepta[1,2-b]thiophen-4-ylidene)-1-piperidinevalerate Hydrochloride

Colorless needles, mp 205-212 °C (dec.) ($EtOH-Et_2O$).

Analysis for $C_{25}H_{29}NO_3S \cdot HCl$:

| | |
|---|---|
| Calculated | C, 65.27; H, 6.57; N, 3.04. |
| Found | C, 65.20; H, 6.52; N, 3.03. |

Example 4
Methyl 4-(4,9-Dihydro-10-oxo-10H-benzo[4,5]cyclohepta[1,2-b]thiophen-4-ylidene)-1-piperidinecaproate Hydrochloride

Colorless needles, mp 200-210 °C (dec.) ($EtOH-Et_2O$).

Analysis for $C_{25}H_{29}NO_3S \cdot HCl \cdot 1/4H_2O$:

| | |
|---|---|
| Calculated | C, 64.64; H, 6.62; N, 3.02. |
| Found | C, 64.73; H, 6.47; N, 2.98. |

Example 5
4-(4,9-Dihydro-10-oxo-10H-benzo[4,5]cyclohepta[1,2-b]thiophen-4-ylidene)-1-piperidinepropionic Acid Hydrochloride

Ethyl acrylate (0.66 g) was added to a suspension of 1.50 g of 4,9-dihydro-4-(4-piperidinylidene)-10H-benzo[4,5]cyclohepta[1,2-b]thiophen-10-one in 7.5 ml of ethanol and the mixture was stirred at 60 °C for 1 hr. After cooling, 6.3 ml of 2N sodium hydroxide aqueous solution was added to the mixture and the solution was stirred for 20 min. The reaction mixture was neutralized with hydrochloric acid and the solvent was removed. Hydrochloric acid was added to the residue. The resulting gum was washed with a little saline and chloroform, and solidified by treatment of acetone to give 1.29 g of colorless crystals, which were recrystallized from a mixture of methanol and ether to give colorless plates, mp 220-225 °C(dec.).

Analysis for $C_{21}H_{21}NO_3S \cdot HCl \cdot 1/4H_2O$:

| | |
|---|---|
| Calculated | C, 61.76; H, 5.55; N, 3.43. |
| Found | C, 61.62; H, 5.56; N, 3.22. |

Example 6
4-(4,9-Dihydro-10-oxo-10H-benzo[4,5]cyclohepta[1,2-b]thiophen-4-ylidene)-1-piperidinebutyric Acid Hydrochloride

2N Sodium hydroxide aqueous solution (4.35 ml) was added to a solution of 1.60 g of ethyl 4-(4,9-dihydro-10-oxo-10H-benzo[4,5]cyclohepta[1,2-b]thiophen-4-ylidene)-1-piperidinebutyrate hydrochloride in 10 ml of methanol and the mixture was stirred at room temperature for 3.5 hrs. The reaction mixture was neutralized with hydrochloric acid and the solvent wad removed. Hydrochloric acid and chloroform was added to the residue and the resulting gum was solidified by treatment of acetone to give 1.38 g of colorless crystals, which were recrystallized from a mixture of methanol and ether to give colorless plates , mp 180-182 °C

Analysis for $C_{22}H_{23}NO_3S \cdot HCl \cdot H_2O$:

| | |
|---|---|
| Calculated | C, 60.61; H, 6.01; N, 3.21. |
| Found | C, 60.87; H, 6.00; N, 3.03. |

The compounds of Example 7 to 9 were prepared in the same manner described in Example 6.

Example 7
4-(4,9-Dihydro-10-oxo-10H-benzo[4,5]cyclohepta[1,2-b]thiophen-4-ylidene)-1-piperidineacetic Acid

Pale yellow needles, mp 157-160 °C(dec.) (EtOH-$H_2O$).

| | |
|---|---|
| Mass spectrum m/z: | 353 ($M^+$). |
| IR spectrum $\nu$ (KBr) $cm^{-1}$: | 1642 ($COO^-$ ,CO) |
| NMR spectrum $\delta$ (DMSO-$d_6$) ppm: | 2.00-3.10(8H, m), 3.19(2H, s), 3.69(1H, d, 7=13.5Hz), 4.25(1H, d, J=13.5Hz), 7.10-7.50(5H, m), 7.92(1H, d, J=5Hz). |

Example 8

4-(4,9-Dihydro-10-oxo-10H-benzo[4,5]cyclohepta[1,2-b]thiophen-4-ylidene)-1-piperidinevaleric Acid Hydrochloride

Pale brown plates, mp 210-215 °C(dec.) (MeOH-$Et_2O$).

| | |
|---|---|
| Mass spectrum m/z: | 395 ($M^+$). |
| IR spectrum ν (KBr) $cm^{-1}$: | 1726(COO), 1652(CO) |
| NMR spectrum δ (DMSO-$d_6$) ppm: | 1.30-1.90(4H, m), 2.25(2H, t, J=6.5Hz), 2.40-3.50(10H, m), 3.67-(1H, d, J=13.5Hz), 4.38(1H, d, J=13.5Hz), 7.10-7.60(5H, m), 7.94-(1H, d, J=5Hz), 11.50(1H, broad). |

Example 9

4-(4,9-Dihydro-10-oxo-10H-benzo[4,5]cyclohepta[1,2-b]thiophen-4-ylidene)-1-piperidinecaproic Acid Hydrochloride

Colorless needles, mp 230-237 °C(dec.) (MeOH-$Et_2O$).

Analysis for $C_{24}H_{27}NO_3S \cdot HCl$:

| | |
|---|---|
| Calculated | C, 64.63; H, 6.33; N, 3.14. |
| Found | C, 64.72; H, 6.28; N, 3.06. |

Example 10

4-(Dibenzo[b,e]thiepin-11(6H)-ylidene)-1-piperidinepropionic Acid Hydrochloride

1) A mixture of 2.00 g of 4-(dibenzo[b,e]thiepin-11(6H)-ylidene)piperidine hydrochloride, 0.8 ml of ethyl acrylate and 0.9 ml of triethylamine in 10 ml of ethanol was refluxed for 2 hrs. The solvent was removed and water was added to the residue. The solution was extracted with ether and the extract was washed with water, dried and concentrated. The residue was purified by column chromatography on silica gel [eluent: chloroform-methanol (50:1)]to give 2.23 g of ethyl 4-(dibenzo[b,e]thiepin-11(6H)-ylidene)-1-piperidinepropionate as yellow liquid.

| | |
|---|---|
| Mass spectrum m/s: | 393 ($M^+$). |
| IR spectrum ν (liq) $cm^{-1}$: | 1736(COO) |
| NMR spectrum δ ($CDCl_3$) ppm: | 1.24(3H, t, J=7Hz), 2.01-2.83(12H, m), 3.37(1H, d, J=13Hz), 4.13(2H, q, J=7Hz), 4.93(1H, d, J=13Hz), 6.87-7.40(8H, m). |

2) A mixture of 1.80 g of ethyl 4-(dibenzo[b,e]thiepin-11(6H)-ylidene)-1-piperidinepropionate and 4.6 ml of 2N sodium hydroxide aqueous solution in 15 ml of methanol was refluxed for 2 hrs. The solvent was removed and water was added to the residue. The solution was acidified with hydrochloric acid and methylene chloride was added to the solution. The resulting crystals were collected by filtration to give pale brown crystals. The filtrate was extracted with methylene chloride and the extract was dried and concentrated. The residue and the crystals obtained above were recrystallized from a mixture of ethanol and ether to give 1.00 g of the titled compound as pale brown crystals, mp 207-209 °C(dec.).

Analysis for $C_{22}H_{23}NO_2S \cdot HCl$:

| | |
|---|---|
| Calculated | C, 65.74; H, 6.02; N, 3.48. |
| Found | C, 65.54; H, 6.06; N, 3.60. |

Example 11

4-(9H-Thioxanthen-9-ylidene)-1-piperidinebutyric Acid Hydrochloride

1) A mixture of 1.60 g of 4-(9H-thioxanthen-9-ylidene) piperidine, 1.45 g of ethyl 4-bromobutyrate and

0.79 g of potassium carbonate in 8 ml of N,N-dimethylformamide was stirred at 70 °C for 3.5 hrs. After cooling, water was added to the reaction mixture and the solution was extracted with ethyl acetate. The extract was washed with water, dried and concentrated. The residue was converted to the hydrochloride in the usual manner to give 1.84 g of ethyl 4-(9H-thioxanthen-9-ylidene)-1-piperidinebutyrate hydrochloride as colorless crystals, which were recrystallized from a mixture of acetone and ether to give colorless needles, mp 163-165 °C.

Analysis for $C_{24}H_{27}NO_2S \cdot HCl \cdot 1/4H_2O$:

| | |
|---|---|
| Calculated | C, 66.34; H, 6.61; N, 3.22. |
| Found | C, 66.42; H, 6.83; N, 3.11. |

2) A mixture of 1.60 g of ethyl 4-(9H-thioxanthen-9-ylidene)-1-piperidinebutyrate hydrochloride, 5.6 ml of 2N sodium hydroxide aqueous solution and 10ml of methanol was refluxed for 1 hr. The solvent was removed and water was added to the residue. The solution was acidified with hydrochloric acid and the resulting crystals were collected by filtration to give 1.39 g of titled compound as colorless crystals, which were recrystallized from a mixture of methanol and ether to give 1.23 g of colorless needles, mp 242-247 °C.

Analysis for $C_{22}H_{23}NO_2S \cdot HCl$:

| | |
|---|---|
| Calculated | C, 65.74; H, 6.02; N, 3.48. |
| Found | C, 65.69; H, 6.15; N, 3.37. |

The compounds of Example 12 to 19 were prepared in the same manners described in Examples 10 and 11.

Example 12

4-(Dibenzo[b,e]thiepin-11(6H)-ylidene)-1-piperidineacetic Acid

1)Ethyl 4-(dibenzo[b,e]thiepin-11(6H)-ylidene)-1-piperidineacetate

Yellow liquid

| | |
|---|---|
| Mass spectrum m/z: | 379 ($M^+$). |
| IR spectrum $\nu$ (liq) $cm^{-1}$: | 1746(COO) |
| NMR spectrum $\delta$ ($CDCl_3$) ppm: | 1.27(3H, t, J=7Hz), 2.00-2.92(8H, m), 3.25(2H, s), 3.39(1H, d, J=13Hz), 4.18(2H, q, J=7Hz), 4.94(1H, d, J=13Hz), 6.92-7.37(8H, m). |

2)4-(Dibenzo[b,e]thiepin-11(6H)-ylidene)-1-piperidineacetic acid

Pale brown crystals, mp 198-200 °C(dec.) (EtOH-$Et_2O$)

Analysis for $C_{21}H_{21}NO_2S$ :

| | |
|---|---|
| Calculated | C, 71.76; H, 6.02; N, 3.99. |
| Found | C, 71.40; H, 5.90; N, 4.27. |

Example 13

4-(Dibenzo[b,e]thiepin-11(6H)-ylidene)-1-piperidinebutyric Acid Hydrochloride

1)Ethyl 4-(dibenzo[b,e]thiepin-11(6H)-ylidene)-1-piperidinebutyrateYellow liquid

| | |
|---|---|
| Mass spectrum m/z: | 407 ($M^+$). |
| IR spectrum $\nu$ (liq) $cm^{-1}$: | 1734(COO) |
| NMR spectrum $\delta$ ($CDCl_3$) ppm: | 1.25(3H, t, J=7Hz), 1.62-2.02(2H, m), 2.02-2.86(12H, m), 3.38(1H, d, J=13Hz), 4.12(2H, q, J=7Hz), 4.94(1H, d, J=13Hz), 6.91-7.44(8H, m). |

2)4-(Dibenzo[b,e]thiepin-11(6H)-ylidene)-1-piperidinebutyric acid hydrochloride
Pale brown crystals, mp 225-227 °C (dec.) ($EtOH-Et_2O$)

Analysis for $C_{23}H_{25}NO_2S \cdot HCl$:

| | |
|---|---|
| Calculated | C, 66.41; H, 6.30; N, 3.37. |
| Found | C, 66.39; H, 6.23; N, 3.58. |

Example 14

4-(Dibenzo[b,e]thiepin-11(6H)-ylidene)-1-piperidinevaleric Acid Hydrochloride
1)Ethyl 4-(dibenzo[b,e]thiepin-11(6H)-ylidene)-1-piperidinevaleratehydrochloride
Colorless needles, mp 173.5-177 °C ($EtOH-Et_2O$)

Analysis for $C_{26}H_{31}NO_2S \cdot HCl$:

| | |
|---|---|
| Calculated | C, 68.18; H, 7.04; N, 3.06. |
| Found | C, 68.31; H, 6.98; N, 3.21. |

2)4-(Dibenzo[b,e]thiepin-11(6H)-ylidene)-1-piperidinevaleric acid hydrochloride
Pale yellow crystals, mp 229-232 °C(dec.) ($EtOH-Et_2O$)

Analysis for $C_{24}H_{27}NO_2S \cdot HCl$:

| | |
|---|---|
| Calculated | C, 67.04; H, 6.56; N, 3.26. |
| Found | C, 66.83; H, 6.69; N, 3.34. |

Example 15

4-(Dibenzo[b,e]thiepin-11(6H)-ylidene)-1-piperidinecaproic Acid Hydrochloride
1)Methyl 4-(dibenzo[b,e]thiepin-11(6H)-ylidene)-1-piperidinecaproate hydrochloride
Colorless needles, mp 227-230 °C ($MeOH-Et_2O$)

Analysis for $C_{26}H_{31}NO_2S \cdot HCl$:

| | |
|---|---|
| Calculated | C, 68.18; H, 7.04; N, 3.06. |
| Found | C, 68.17; H, 7.07; N, 3.15. |

2)4-(Dibenzo[b,e]thiepin-11(6H)-ylidene)-1-piperidinecaproic acid hydrochloride
Colorless crystals, mp 235-238 °C (dec.) ($EtOH-Et_2O$)

Analysis for $C_{25}H_{29}NO_2S \cdot HCl$:

| | |
|---|---|
| Calculated | C, 67.62; H, 6.81; N, 3.15. |
| Found | C, 67.59; H, 6.76; N, 3.26. |

Example 16

4-(Dibenzo[b,e]thiepin-11(6H)-ylidene)-1-piperidineheptanoic Acid Hydrochloride
1)Methyl 4-(dibenzo[b,e]thiepin-11(6H)-ylidene)-1-piperidineheptanoate hydrochloride
Colorless needles, mp 190-194 °C (MeOH-$Et_2O$)

Analysis for $C_{27}H_{33}NO_2S \cdot HCl$:

| | |
|---|---|
| Calculated | C, 68.69; H, 7.26; N, 2.97. |
| Found | C, 68.78; H, 7.19; N, 3.06. |

2)4-(Dibenzo[b,e]thiepin-11(6H)-ylidene)-1-piperidineheptanoic acid hydrochloride
Colorless crystals, mp 243-245 °C (dec.) (EtOH-$Et_2$ O)

Analysis for $C_{26}H_{31}NO_2S \cdot HCl$:

| | |
|---|---|
| Calculated | C, 68.18; H, 7.04; N, 3.06. |
| Found | C, 68.28; H, 7.00; N, 3.15. |

Example 17

4-(Dibenzo[b,e]thiepin-11(6H)-ylidene)-1-piperidineoctanoic Acid Hydrochloride
1)Methyl 4-(dibenzo[b,e]thiepin-11-(6H)-ylidene)-1-piperidineoctanoate hydrochloride
Colorless needles, mp 201-205 °C (MeOH-$Et_2O$)

Analysis for $C_{28}H_{35}NO_2S \cdot HCl$:

| | |
|---|---|
| Calculated | C, 69.18; H, 7.46; N, 2.88. |
| Found | C, 69.34; H, 7.45; N, 2.95. |

2)4-(Dibenzo[b,e]thiepin-11(6H)-ylidene)-1-piperidineoctanoic acid hydrochloride
Colorless crystals, mp 242-245 °C (dec.) (EtOH-$Et_2$ O)

Analysis for $C_{27}H_{33}NO_2S \cdot HCl$:

| | |
|---|---|
| Calculated | C, 68.69; H, 7.26; N, 2.97. |
| Found | C, 68.55; H, 7.25; N, 3.09. |

Example 18

4-(9H-Thioxanthen-9-ylidene)-1-piperidinepropionic Acid 1)Ethyl 4-(9H-thioxanthen-9-ylidene)-1-piperidinepropionate hydrochloride
Colorless plates, mp 185-188 °C ($Me_2CO$-$Et_2O$)

Analysis for $C_{23}H_{25}NO_2S \cdot HCl \cdot 1/4H_2O$:

| | |
|---|---|
| Calculated | C, 65.70; H, 6.35; N, 3.33. |
| Found | C, 65.90; H, 6.39; N, 3.13. |

2)4-(9H-Thioxanthen-9-ylidene)-1-piperidinepropionic acid
Pale yellow needles, mp 233-236 °C (dec.) (DMF-$H_2O$)

Analysis for $C_{21}H_{21}NO_2S \cdot 9/4H_2O$:

| | |
|---|---|
| Calculated | C, 64.34; H, 6.56; N, 3.57. |
| Found | C, 64.56; H, 6.29; N, 3.38. |

Example 19

4-(9H-Thioxanthen-9-ylidene)-1-piperidinevaleric Acid 1)Ethyl 4-(9H-thioxanthen-9-ylidene)-1-piperidinevalerate fumarate
Colorless needles, mp 203-205 °C (EtOH)

Analysis for $C_{25}H_{29}NO_2S \cdot C_4H_4O_4$:

| | |
|---|---|
| Calculated | C, 66.52; H, 6.35; N, 2.67. |
| Found | C, 66.47; H, 6.28; N, 2.54. |

2)4-(9H-Thioxanthen-9-ylidene)-1-piperidinevaleric acid
Colorless needles, mp 204-207 °C (dec.) (DMF-$H_2O$)

Mass spectrum m/z: 379 ($M^+$).
IR spectrum $\nu$ (KBr) $cm^{-1}$: 1580($COO^-$)
NMR spectrum $\delta$ (DMSO-$d_6$) ppm: 1.20-1.80(4H, m), 1.90-3.00(12H, m), 7.00-7.80(8H, m).

Example 20

tert-Butyl 4-(4,9-Dihydro-10-oxo-10H-benzo[4,5]cyclohepta[1,2-b]thiophen-4-ylidene)-1-piperidinepropionate

A mixture of 12.6 g of 4,9-dihydro-4-(4-piperidinylidene)-10H-benzo[4,5]cyclohepta[1,2-b]thiophen-10-one and 18 ml of tert-butyl acrylate in 80 ml of acetonitrile was refluxed for 5 hrs and the solvent was removed. The resulting residue was purified by column chromatography on silica gel (eluent: methylene chloride) to give 16.3 g of pale brown viscous liquid.

Mass spectrum m/z: 423 ($M^+$).
IR spectrum $\nu$ (liq) $cm^{-1}$: 1730 (COO), 1660 (CO).
NMR spectrum $\delta$ ($CDCl_3$) ppm: 1.45(9H, s), 2.05-2.95(12H, m), 3.75(1H, d, J=13.5 Hz), 4.21(1H, d, J=13.5Hz), 7.02(1H, d, J=5Hz), 7.10-7.35(4H, m), 7.53(1H, d, J=5Hz).

Example 21

4-(4,9-Dihydro-10-oxo-10H-benzo[4,5]cyclohepta[1,2-b]thiophen-4-ylidene)-1-piperidinepropionic Acid Hydrochloride

Hydrochloric acid (5.7 ml) was added by portions to a solution of 6.57 g of tert-butyl 4-(4,9-dihydro-10-oxo-10H-benzo[4,5]cyclohepta[1,2-b]thiophen-4-ylidene)-1-piperidinepropionate in 23 ml of acetonitrile and the mixture was stirred at 60 to 70 °C for 30 min. The resulting crystals were collected by filtration to give 5.70 g of colorless crystals.

This compound was identified by comparing its mp, IR spectrum and NMR spectrum with those of the compound of Example 5.

Example 22

Ethyl 4-(4,9-Dihydro-10-oxo-10H-benzo[4,5]cyclohepta[1,2-b]thiophen-4-ylidene)-1-piperidinepropionate

A mixture of 9.44 g of 4,9-dihydro-4-(4-piperidinylidene)-10H-benzo[4,5]cyclohepta[1,2-b]thiophen-10-one and 4 ml of ethyl acrylate in 50 ml of ethanol was refluxed for 2 hrs and the solvent was removed. The resulting residue was purified by column chromatography on silica gel (eluent: ethyl acetate) to give 10.3 g of pale brown viscous liquid.

| | |
|---|---|
| Mass spectrum m/z: | 395 ($M^+$). |
| IR spectrum $\nu$ (liq) $cm^{-1}$: | 1734 (COO), 1658 (CO). |
| NMR spectrum $\delta$ ($CDCl_3$) ppm: | 1.26(3H, t, J=7Hz), 2.05-2.90(12H, m), 3.75 (1H, d, J=13.5Hz), 4.14-(2H, q, J=7Hz), 4. 21(1H, d, J=13.5Hz), 7.02(1H, d, J=5Hz), 7.10-7.35(4H, m), 7.53 (1H, d, J=5Hz). |

Example 23

Tablets of a pharmaceutical preparation according to the present invention are prepared in the usual manner using the following constituents:

| | |
|---|---|
| Compound of the present invention | 10 mg |
| Lactose | q.s. |
| Corn starch | 34 mg |
| Magnesium stearate | 2 mg |
| Hydroxypropylmethylcellulose | 8 mg |
| Polyethyleneglycol 6000 | 0.5 mg |
| Titanium oxide | 0.5 mg |
| | 120 mg |

Example 24

Capsules of a pharmaceutical preparation according to the present invention are prepared in the usual manner using the following constituents:

| | | |
|---|---|---|
| Compound of the present invention | 10 | mg |
| Lactose | q.s. | |
| Calcium carboxymethylcellulose | 15 | mg |
| Hydroxypropylcellulose | 2 | mg |
| Magnesium stearate | 2 | mg |
| | 100 | mg |

Example 25

Powders of a pharmaceutical preparation according to the present invention are prepared in the usual manner using the following constituents:

| | | |
|---|---|---|
| Compound of the present invention | 20 | mg |
| Lactose | q.s. | |
| D-Mannitol | 500 | mg |
| Hydroxypropylcellulose | 5 | mg |
| Talc | 2 | mg |
| | 1000 | mg |

Example 26

Injections of a pharmaceutical preparation according to the present invention are prepared in the usual manner using the following constituents:

| | | |
|---|---|---|
| Compound of the present invention | 1 | mg |
| Glucose | 50 | mg |
| Hydrochloric acid | q.s. | |
| Distilled water for injection | q.s. | |
| | 2 | ml |

Example 27

Suppositories of a pharmaceutical preparation according to the present invention are prepared in the usual manner using the following constituents:

| | | |
|---|---|---|
| Compound of the present invention | 5 | mg |
| Hard fat | 1295 | mg |
| | 1300 | mg |

Example 28

Plasters of a pharmaceutical preparation according to the present invention are prepared in the usual manner using the following constituents:

| | | |
|---|---|---|
| Compound of the present invention | 10 | mg |
| Gelatin | 1100 | mg |
| Polyvinylalcohol | 250 | mg |
| Methylcellulose | 100 | mg |
| Glycerin | 1500 | mg |
| Sodium polyacrylate | 50 | mg |
| Polybutene | 150 | mg |
| Purified water | 990 | mg |
| | 5000 | mg |

## Claims

1. A piperidine compound represented by the following formula (I):

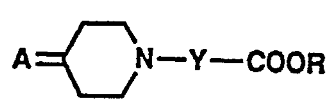

(I)

wherein Y represents an alkylene group having 1 to 7 carbon atoms; A is a group represented by the following formula I-a:

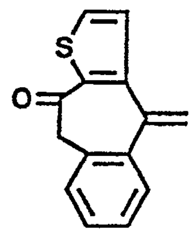

(I-a)

or A is a group represented by the following formula I-b:

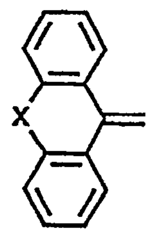

(I-b)

wherein X represents $-CH_2$ -S- or -S-; and R represents a hydrogen atom or a lower alkyl group with a proviso that A is a group represented by formula I-a, or R represents a hydrogen atom with a proviso that A is a group represented by formula I-b; and a pharmacologically acceptable salt thereof.

**2.** 4-(4,9-Dihydro-10-oxo-10H-benzo[4,5]cyclohepta[1,2-b]thiophen-4-ylidene)-1-piperidinepropionic acid and a pharmacologically acceptable salt thereof.

**3.** 4-(4,9-Dihydro-10-oxo-10H-benzo[4,5]cyclohepta[1,2-b]thiophen-4-ylidene)-1-piperidinevaleric acid and a pharmacologically acceptable salt thereof.

**4.** 4-(Dibenzo[b,e]thiepin-11(6H)-ylidene)-1-piperidineheptanoic acid and a pharmacologically acceptable salt thereof.

**5.** 4-(Dibenzo[b,e]thiepin-11(6H)-ylidene)-1-piperidineoctanoic acid and a pharmacologically acceptable salt thereof.

**6.** 4-(9H-Thioxanthen-9-ylidene)-1-piperidinepropionic acid and a pharmacologically acceptable salt thereof.

**7.** A method for preparing a piperidine compound represented by the following formula (I):

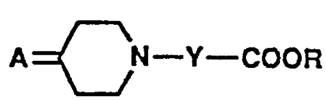

(I)

wherein Y represents an alkylene group having 1 to 7 carbon atoms; A is a group represented by the following formula I-a:

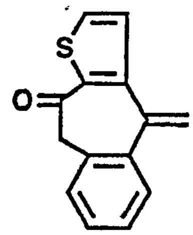

(I-a)

or A is a group represented by the following formula I-b:

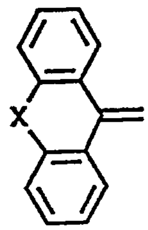

(I-b)

wherein X represents $-CH_2-S-$ or -S-; and R represents a hydrogen atom or a lower alkyl group with a proviso that A is a group represented by formula I-a, or R represents a hydrogen atom with a proviso that A is a group represented by formula I-b; and a pharmacologically acceptable salt thereof, comprising the steps of reacting in a solvent or without a solvent in the presence or absence of a base as dehydrohalogenating agent a compound represented by the following general formula (II):

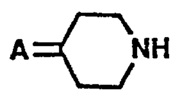

(II)

wherein A is the same as that defined above, with a compound represented by the following formula (III):

Z-Y-COOR (IIIa) or $CH_2 = CH-COOR$ (IIIb)

wherein Y and R are the same as those defined above, and Z represents a halogen atom, and followed by hydrolysis by using an acid or a base, if necessary.

8. A pharmaceutical composition comprising an effective amount of a piperidine compound represented by the following formula (I):

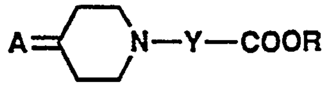

(I)

wherein Y represents an alkylene group having 1 to 7 carbon atoms; A is a group represented by the following formula I-a:

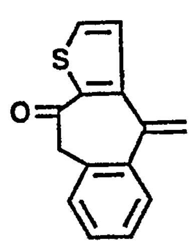

(I-a)

or A is a group represented by the following formula I-b:

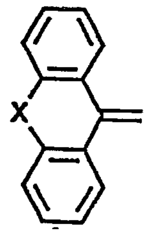

(I-b)

wherein X represents $-CH_2$ -S- or -S-; and R represents a hydrogen atom or a lower alkyl group with a proviso that A is a group represented by formula I-a, or R represents a hydrogen atom with a proviso that A is a group represented by formula I-b; or a pharmacologically acceptable salt thereof, together with a pharmaceutically acceptable carrier or coating.

**9.** The pharmaceutical composition according to claim 9, which is useful for the treatment of allergic diseases.

**10.** The pharmaceutical composition according to claim 9, which is useful for the treatment of bronchial asthma.

**11.** An antihistaminic agent comprising an effective amount of a piperidine compound represented by the following formula (I):

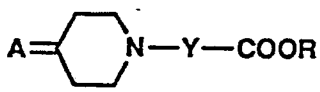

(I)

wherein Y represents an alkylene group having 1 to 7 carbon atoms; A is a group represented by the following formula I-a:

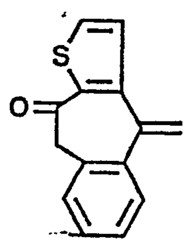

(I-a)

or A is a group represented by the following formula I-b:

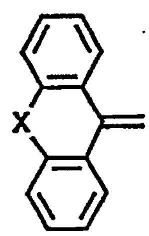

(I-b)

wherein X represents $-CH_2$ -S- or -S-; and R represents a hydrogen atom or a lower alkyl group with a proviso that A is a group represented by formula I-a, or R represents a hydrogen atom with a proviso that A is a group represented by formula I-b; or a pharmacologically acceptable salt thereof.

**12.** An antiallergic agent comprising an effective amount of a piperidine compound represented by the

following formula (I):

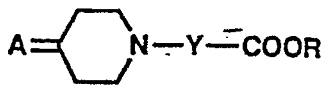

(I)

wherein Y represents an alkylene group having 1 to 7 carbon atoms; A is a group represented by the following formula I-a:

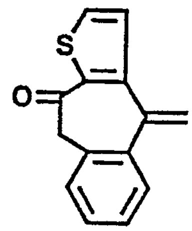

(I-a)

or A is a group represented by the following formula I-b:

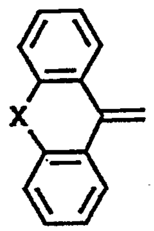

(I-b)

wherein X represents $-CH_2$ -S- or -S-; and R represents a hydrogen atom or a lower alkyl group with a proviso that A is a group represented by formula I-a, or R represents a hydrogen atom with a proviso that A is a group represented by formula I-b; or a pharmacologically acceptable salt thereof.

**13.** An agent for bronchial asthma comprising an effective amount of a piperidine compound represented by the following formula (I):

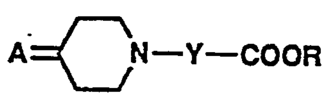

(I)

wherein Y represents an alkylene group having 1 to 7 carbon atoms; A is a group represented by the following formula I-a:

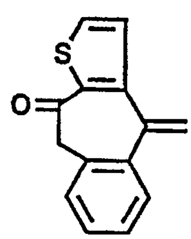

(I-a)

or A is a group represented by the following formula I-b:

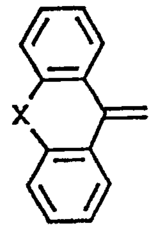

(I-b)

wherein X represents $-CH_2-S-$ or -S-; and R represents a hydrogen atom or a lower alkyl group with a proviso that A is a group represented by formula I-a, or R represents a hydrogen atom with a proviso that A is a group represented by formula I-b; or a pharmacologically acceptable salt thereof.

**14.** A method for the treatment of an allergic disease comprising the step of administering to a mammal an effective amount of a substance selected from the group consisting essentially of (a) a piperidine compound represented by the following formula (I):

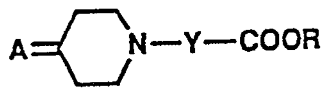

(I)

wherein Y represents an alkylene group having 1 to 7 carbon atoms; A is a group represented by the following formula I-a:

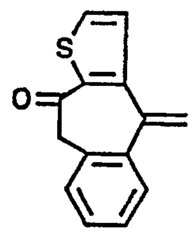

(I-a)

or A is a group represented by the following formula I-b:

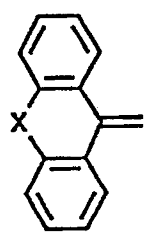

(I-b)

wherein X represents $-CH_2$ -S- or -S-; and R represents a hydrogen atom or a lower alkyl group with a proviso that A is a group represented by formula I-a, or R represents a hydrogen atom with a proviso that A is a group represented by formula I-b; or a pharmacologically acceptable salt thereof; (b) an antiallergic agent comprising the compounds of formula (I); and a pharmaceutical composition comprising the compounds of formula (I).

**15.** A method for the treatment of an allergic disease according to claim 15, wherein said step of administering is performed on a human being.

**16.** A method for the treatment of bronchial asthma comprising the step of administering to a mammal an effective amount of a substance selected from the group consisting essentially of (a) a piperidine compound represented by the following formula (I):

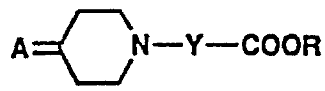

(I)

wherein Y represents an alkylene group having 1 to 7 carbon atoms; A is a group represented by the following formula I-a:

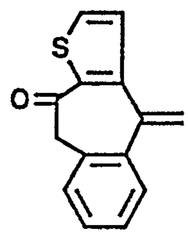

(I-a)

or A is a group represented by the following formula I-b:

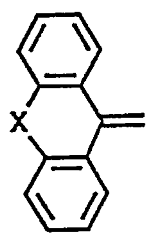

(I-b)

wherein X represents $-CH_2-S-$ or -S-; and R represents a hydrogen atom or a lower alkyl group with a proviso that A is a group represented by formula I-a, or R represents a hydrogen atom with a proviso that A is a group represented by formula I-b; or a pharmacologically acceptable salt thereof; (b) an agent for bronchial asthma comprising the compounds of formula (I); and a pharmaceutical composition comprising the compounds of formula (I).

**17.** A method for the treatment of bronchial asthma according to claim 17, wherein said step of administering is performed on a human being.

European Patent Office

# PARTIAL EUROPEAN SEARCH REPORT

which under Rule 45 of the European Patent Convention shall be considered, for the purposes of subsequent proceedings, as the European search report

Application number EP 91105567

- page 1 -

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-2256392 (SANDOZ AG)<br>* claims; page 8, examples 6,9; page 10, example 15; page 3, line 14 - page 4, line 31 * | 1,7-13 | C07D409/04<br>A61K31/445<br>//(C07D409/04, 333:00,211:00)<br>(C07D409/04, 335:00,211:00)<br>(C07D409/04, 337:00,211:00) |
| Y | FR-M-3138 (SPOFA)<br>* page 4, column 2; abstract * | 1,9-13 | |
| Y | EP-A-0042544 (SCHERING CORP.)<br>* abstract; examples 1,2 * | 1,9-13 | |
| Y | US-A-4072756 (A. EBNÖTHER et al.)<br>* examples 1,2; column 15, lines 6-17 * | 1,7,10,13 | |
| Y | DE-A-2111071 (SANDOZ AG)<br>* all examples; page 7, lines 29-39 * | 1,7-13 | |
| | ... | | |

TECHNICAL FIELDS SEARCHED (Int. Cl.5)

C07D221/00
C07D333/00
C07D335/00
C07D337/00
C07D409/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-13

Claims searched incompletely: -

Claims not searched: 14-17

Reason for the limitation of the search:

Article 52(4) EPC

| Place of search | Date of completion of the search | Examiner |
|---|---|---|
| Berlin | 15.07.1991 | D. FRELON |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

European Patent Office

# PARTIAL EUROPEAN SEARCH REPORT

Application number
EP 91105567


| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| Y | WO-A-8910363 (SCHERING CORP.)<br>* example 2, page 83; pages 69-76 * | 1,7-13 | |
| A,D | HELVETICA CHIMICA ACTA<br>vol. 59, no. 3, 1976, pages 866-877;<br>E. WALDVOGEL et al.: "Untersuchungen über synthetische Arzneimittel 9- und 10-Oxo-Derivate von 9,10-Dihydro-4H-benzo(4,5)cyclohepta-(1,2-b)thiophenen"<br>* pages 866,867: "Einleitung" * | 9-13 | |
| | | | TECHNICAL FIELDS SEARCHED (Int. Cl.5) |